# FASCICULE DE BREVET EUROPEEN

(11) **EP 1 438 095 B1**
(45) Date de publication et mention de la délivrance du brevet: **15.04.2009**
(21) Numéro de dépôt: 02800179.0
(22) Date de dépôt: 03.10.2002
(51) Int. Cl.: A61M 15/00, G06M 1/24

(54) **COMPTEUR DE DOSES POUR DISTRIBUTEUR DE PRODUIT FLUIDE**
DOSIMETER FÜR EINEN SPENDER FÜR EIN FLÜSSIGES PRODUKT
DOSIMETER FOR FLUID PRODUCT DISPENSER

(30) Priorité: 04.10.2001 FR 0112771
(43) Date de publication de la demande: 21.07.2004
(73) Titulaire: Valois SAS, 27110 Le Neubourg (FR)
(72) Inventeur: BRUNA, Pascal, F-76300 Sotteville les Rouen (FR)
(74) Mandataire: CAPRI
(86) Numéro de dépôt international: PCT/FR2002/003376
(87) Numéro de publication internationale: WO 2003/028792

(56) Documents cités:
- EP-A- 0 684 047
- EP-A- 0 949 584
- WO-A-01/37909
- WO-A-98/52634
- FR-A- 637 259
- FR-A- 2 799 858
- US-A- 3 713 582

## Description

La présente invention concerne un compteur de doses pour distributeur de produit fluide, et un distributeur de produit fluide incorporant un tel compteur.

Un compteur de doses pour distributeur de produit fluide selon le préambule de la revendication 1 est connu du document FR-A-2 799 858.

Dans le domaine des distributeurs de produit fluide, et en particulier dans le domaine de la pharmacie, il est connu d'utiliser un compteur de doses pour déterminer le nombre de doses distribué ou restant à distribuer dans le distributeur. Ces compteurs sont généralement actionnés lors de l'utilisation du distributeur, de sorte qu'ils sont adaptés à compter à chaque fois qu'une dose de produit est expulsée par le distributeur. La présence d'un compteur de doses dans un distributeur de produit fluide, qu'il s'agisse d'un distributeur à pompe ou d'un distributeur à valve doseuse, implique une modification structurelle relativement importante du distributeur pour permettre l'adaptation du compteur. Il s'en suit une complication de fabrication et de montage, et donc une augmentation du coût pour l'appareil en question. Un autre problème qui se pose avec les compteurs existants est la difficulté de pouvoir compter un grand nombre de doses, ce qui implique généralement une augmentation de dimension assez importante du compteur, ce qui peut rendre son adaptation dans un distributeur de produit fluide compliquée, voire impossible. D'autre part, les distributeurs de produit fluide pouvant être très différents les uns des autres, notamment selon la nature de l'organe de distribution utilisé, à savoir une valve doseuse ou une pompe, il est souvent difficile d'adapter un même compteur à ces différents dispositifs, de sorte qu'il est souvent nécessaire de fabriquer un nouveau compteur spécifique à chaque distributeur, ce qui augmente considérablement le coût de développement et de fabrication du dispositif.

La présente invention a pour but de fournir un compteur de doses pour distributeur de produit fluide qui ne reproduit pas les inconvénient susmentionnés.

La présente invention a notamment pour but de fournir un compter doses qui soit simple et peu coûteux à fabriquer et à assembler.

La présente invention a également pour but de fournir un compteur de doses qui soit compact et de faibles dimensions tout en permettant le comptage d'un grand nombre de doses.

La présente invention a également pour but de fournir un compteur de doses qui s'adapte très facilement à tous les types de dispositifs de distribution de produit fluide connus.

La présente invention a aussi pour but de fournir un compteur de doses qui soit fiable, ergonomique, et facilement visible par l'utilisateur du distributeur de produit fluide sur lequel le compteur est monté.

La présente invention a donc pour objet un compteur de doses pour distributeur de produit fluide, comportant deux disques de comptage superposés, un premier disque des unités et un second disque des dizaines et/ou centaines, chaque disque étant monté rotatif autour d'un axe de rotation, lesdits axes de rotation étant parallèles et décalés l'un par rapport à l'autre, caractérisé en ce que ledit compteur comporte un couvercle, ledit couvercle comportant l'axe de rotation du premier disque et l'axe de rotation du second disque.

Avantageusement, le second disque des dizaines et/ou centaines est au moins partiellement transparent et est disposé au-dessus du premier disque des unités dans le sens d'observation de l'utilisateur.

Avantageusement, le couvercle est disposé au-dessus des disques de comptage dans le sens d'observation de l'utilisateur, ledit couvercle incorporant un logement périphérique recevant le second disque des dizaines et/ou centaines et étant pourvu d'une fenêtre de visualisation pour l'utilisateur, l'axe de rotation du premier disque étant décalé par rapport à l'axe du couvercle formant axe de rotation du second disque.

Avantageusement, les premier et second disques de comptage comportent des moyens anti-retour respectifs permettant une rotation seulement dans le sens de comptage.

Avantageusement, les moyens anti-retour du premier disque des unités sont formés par un cran radial du couvercle coopérant avec des dents radiales périphériques dudit premier disque.

Avantageusement, les moyens anti-retour du second disque des dizaines et/ou centaines sont formés par une projection axiale du couvercle coopérant avec des évidements axiaux périphériques dudit second disque.

De préférence, le premier disque est entraîné en rotation à chaque actionnement du distributeur de produit fluide et le second disque est entraîné en rotation par le premier disque à chaque tour complet de celui-ci.

Avantageusement, le compteur est décroissant et compte le nombre de doses restantes dans le distributeur de produit fluide.

Avantageusement, sont prévus des moyens indicateurs de fin de comptage.

En particulier, lesdits moyens indicateurs sont prévus sur le second disque des dizaines et/ou centaines, et sont réalisés sous la forme d'une partie partiellement et/ou totalement opaque.

La présente invention a également pour but un distributeur de produit fluide comportant un compteur tel que défini ci-dessus.

De préférence, le distributeur comporte un élément d'actionnement adapté à actionner le premier disque de comptage à chaque actionnement dudit distributeur.

D'autres caractéristiques et avantages de la présente invention apparaîtront plus clairement au cours de la description détaillée suivante d'un mode de réalisation de celle-ci, faite en relation aux dessins joints, donnés à titre d'exemple non limitatif, et sur lesquels,
- la figure 1 est une vue schématique explosée d'un compteur selon un mode de réalisation de la présente invention,
- la figure 2 est une vue schématique partiellement explosée d'un distributeur de produit fluide incorporant un compteur selon un mode de réalisation de la présente invention,
- la figure 3 est une vue schématique en perspective montrant la coopération entre le couvercle et le premier disque des unités du compteur de la figure 1,
- la figure 4 est une vue similaire à celle de la figure 3, montrant la coopération entre le couvercle et le second disque des dizaines et/ou centaines,
- les figures 5 à 8 montrent une séquence d'actionnement du premier disque des unités,
- la figure 9 est une vue schématique de dessus du compteur selon un mode de réalisation de la présente invention, et
- les figures 10 et 11 montrent des vues similaires à celle de la figure 9 montrant le fonctionnement des moyens indicateurs de fin de comptage.

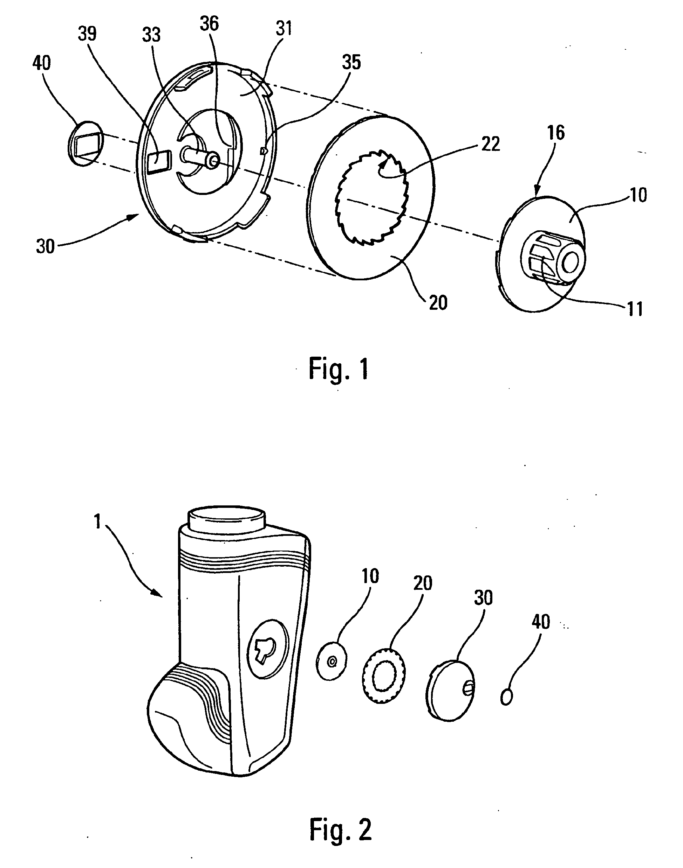

L'exemple de réalisation représenté sur les dessins est un compteur qui s'applique plus particulièrement à un dispositif appelé MDI (inhalateur de doses mesurées), comme cela est représenté sur la figure 2. Il est toutefois entendu que la présente invention ne se limite pas à ce type de dispositif de distribution, et qu'elle s'applique au contraire à tous types de distributeurs de produit fluide, du moment que celui-ci comporte un élément mobile lors de l'actionnement qui permet d'actionner le compteur. Par exemple, tout distributeur comportant un réservoir et une pompe montée sur le réservoir peut recevoir un compteur tel que décrit dans la présente invention, l'élément d'actionnement qui coopère avec le compteur pouvant être une partie quelconque qui se déplace lors de l'actionnement, par exemple la tête de distribution, le poussoir, le réservoir, ou un élément solidaire de celui-ci, etc. Dans l'exemple représenté sur la figure 2, l'élément qui actionne le compteur est un élément d'actionnement 5 (non visible sur la figure 2) qui est généralement solidaire du réservoir, ce dernier étant enfoncé dans le corps du dispositif pour distribuer une dose de produit.

La description du compteur va donc être faite, de manière plus générale, en référence aux figures, et sans être spécifiquement dirigée sur le distributeur MDI représenté sur la figure 2. Celui-ci est toutefois illustratif à titre d'exemple, et il permet de montrer en particulier que le compteur peut être utilisé de manière verticale dans une paroi latérale du dispositif. Ceci est également un mode de réalisation parmi d'autres possibles, et le compteur de l'invention s'applique tout à fait dans une position différente de celle de la figure 2.

Le compteur de doses pour distributeur de produit fluide comporte deux disques de comptage 10 et 20, qui sont superposés axialement l'un sur l'autre. Le premier disque de comptage 10 est le disque des unités, et il est par conséquent adapté à faire un tour complet en dix rotations partielles de 36°. Le second disque de comptage est le disque des dizaines et/ou centaines 20. Une particularité du compteur de l'invention est que ces deux disques de comptage tournent autour d'axes de rotation qui sont parallèles mais décalés l'un par rapport à l'autre. Ceci permet en particulier de réaliser un disque des unités 10 nettement plus petit que le disque des dizaines et/ou centaines 20, tout en assurant une bonne visualisation du nombre de doses compté.

Selon l'invention, le compteur comporte un couvercle 30 qui forme à la fois l'axe de rotation 33 du premier disque 10 et l'axe de rotation 23 du second disque.

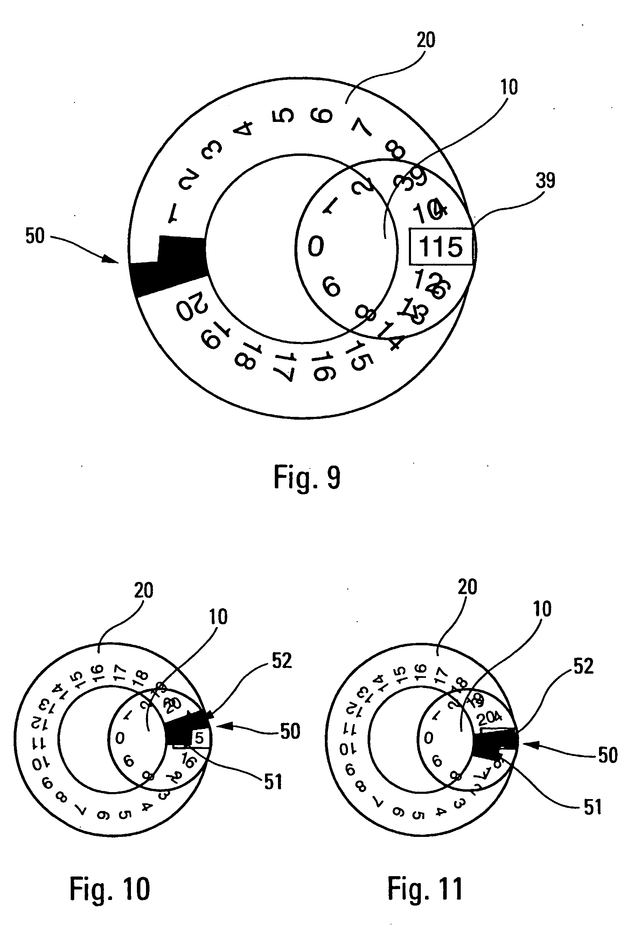

De préférence, le second disque des dizaines et/ou centaines 20 est au moins partiellement transparent et est disposé au-dessus du premier disque des unités 10 dans le sens d'observation de l'utilisateur. De cette manière, l'unité est visible par transparence comme cela est clairement visible sur la figure 9 qui montre le chiffre compté, à savoir le chiffre 115, qui apparaît dans une fenêtre de visualisation 39 du compteur.

Avantageusement, le couvercle 30 incorpore cette fenêtre de visualisation 39. Le couvercle 30 est adapté à recevoir le second disque des dizaines et/ou centaines 20 dans un logement 31 prévu à cet effet, ledit second disque 20 tournant autour de l'axe central dudit couvercle, qui forme donc l'axe de rotation 23 dudit second disque 20. Comme expliqué ci-dessus, le couvercle 30 incorpore également l'axe 33 sur lequel est assemblé le premier disque de comptage 10, ledit axe 33 étant décalé par rapport à l'axe 23 du second disque. Ce décalage apparaît particulièrement clairement sur les figures. Avantageusement, c'est le premier disque 10, une fois monté sur l'axe 33, qui maintient le second disque 20 en place dans le logement 31.

Avantageusement, le compteur comporte des moyens anti-retour pour le premier disque de comptage 10 et pour le second disque de comptage 20. Dans l'exemple représenté sur les figures, ces moyens anti-retour sont de préférence formés sur le couvercle 30. Ainsi, les premiers moyens anti-retour du premier disque des unités 10 sont avantageusement formés par un cran radial 36 dans le couvercle qui coopère avec des dents radiales périphériques 16 dudit premier disque 10. Les seconds moyens anti-retour du second disque des dizaines et/ou centaines 20 sont avantageusement formés par une projection axiale 35 du couvercle 30 qui coopère avec des évidements axiaux périphériques correspondants 25 dudit second disque 20. Dans cette variante, ces seconds moyens anti-retour 25, 35 n'empêchent donc pas véritablement une rotation du second disque 20 dans le sens inverse à celui qui lui est imparti lors de l'actionnement du compteur, mais ils empêchent tout déplacement accidentel du second disque 20, pendant le stockage ou le transport ou pendant tout utilisation autre que la distribution d'une dose de produit par le distributeur. La projection axiale 35 du couvercle pénètre dans un évidement respectif du disque 20 et une certaine force est nécessaire pour permettre à ladite projection axiale 35 de sortir dudit évidement axial 25, permettant ainsi une rotation du second disque 20, ladite projection axiale 35 venant alors coopérer avec l'évidement axial 25 qui suit sur la périphérie du disque 20.

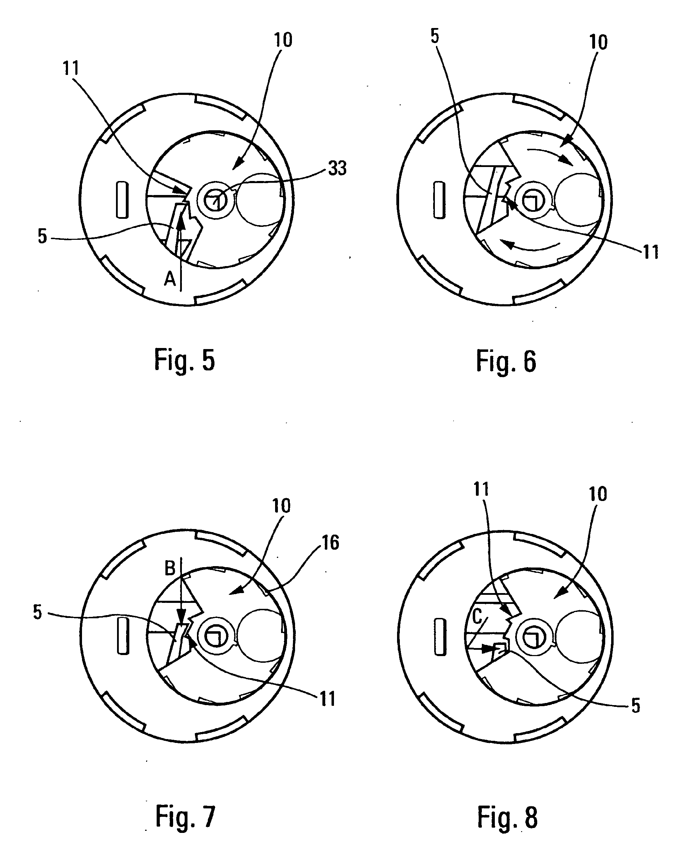

Les figures 5 à 8 montrent un cycle d'actionnement du premier disque des unités 10 au moyen d'un élément d'actionnement 5 solidaire du distributeur de produit fluide. Ces figures 5 à 8 sont partiellement découpées pour permettre la visualisation dudit élément d'actionnement 5. Le premier disque d'unités 10 comporte une série de dix dents périphériques 11 plus clairement visibles sur la figure 1. Lorsque l'utilisateur actionne le dispositif, l'élément d'actionnement 5 vient coopérer avec une dent 11 et, se déplaçant selon la flèche A sur la figure 5, fait tourner ledit premier disque 10 autour de son axe de rotation 33. La figure 5 montre la fin d'actionnement de l'élément d'actionnement 5, avec le disque 10 ayant tourné d'un angle de 36° correspondant à une unité comptée. Lorsque le distributeur de produit fluide revient vers sa position de repos, l'élément d'actionnement 5 revient également vers sa position initiale, ce qui est représenté par la flèche B sur la figure 7. L'élément d'actionnement 5 glisse alors sur lesdites dents 11, et le premier disque des unités 10 est empêché de tourner en sens inverse par les premiers moyens anti-retour formés ici par les dents radiales 16 du premier disque des unités 10 coopérant avec le cran radial 36 du couvercle (non représenté sur ces figures). A fin du cycle, l'élément d'actionnement 5 vient s'encliqueter sous la prochaine dent du premier disque, comme cela est représenté par la flèche C sur la figure 8. Le dispositif est alors prêt pour un cycle d'actionnement ultérieur.

A chaque tour complet du premier disque des unités 10, le second disque 20 est entraîné en rotation. La valeur de cette rotation est dépendante du nombre de doses à compter au moyen de ce second disque 20. Dans l'exemple représenté sur les figures, notamment les figures 9 à 11, le second disque de comptage comporte vingt positions de comptage, ce qui permet donc de compter deux cents doses. Bien entendu, un nombre quelconque de doses peut être compté au moyen du compteur de la présente invention, en modifiant les chiffres inscrits sur le second disque 20. Pour faire tourner le second disque de comptage 20 à chaque tour complet du premier disque des unités 10, ce dernier comporte une dent d'entraînement 12 qui vient coopérer avec des dents 22 du second disque 20. En particulier, cette dent d'actionnement 12 est mise en contact avec les dents 22 du second disque de comptage 20 une seule fois à chaque tour complet du fait du décalage entre les axes de rotation des deux disques 10 et 20. En variante, on peut prévoir une surface de came ou tout autre moyen similaire, qui permet de solidariser les deux disques à chaque tour complet du premier disque 10.

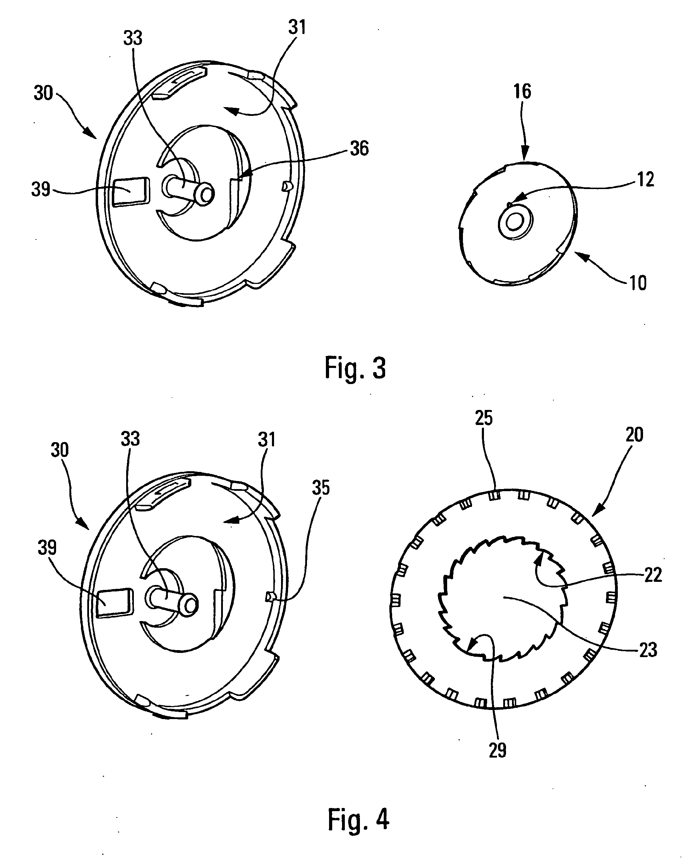

De préférence, le compteur comporte également des moyens indicateurs de fin de comptage 50. Ceux-ci sont représentés sur les figures 10 et 11. Ainsi, dans le mode de réalisation représenté sur ces dessins, le compteur est décroissant et est adapté à compter le nombre de doses restantes dans le dispositif, c'est à dire qu'il décompte le nombre de doses à chaque actionnement. Les moyens indicateurs de fin de comptage 50 comportent alors avantageusement une première partie 51 et une seconde partie 52. La première partie 51 des moyens indicateurs 50 est adaptée à ne permettre une visualisation que des chiffres inscrits sur le premier disque de comptage des unités 10, de sorte que lorsqu'il ne reste plus que dix doses à distribuer, l'utilisateur est prévenu par la présence de ladite première partie 51 des moyens indicateurs 50 dans la fenêtre de visualisation 39. Lorsque la dernière dose à été distribuée, la seconde partie 52 des moyens indicateurs 50 vient obturer complètement la fenêtre de visualisation 39, et l'utilisateur sait alors qu'il n'y a plus de doses à distribuer, ou que le nombre de doses prévu à l'origine a été distribué. Avantageusement, ces moyens indicateurs 50 sont réalisés par des parties partiellement et/ou totalement opaques du second disque de comptage 20, comme visible sur les figures 9 à 11. Pour éviter que des actionnements ultérieurs ne fassent tourner le second disque en libérant ainsi la fenêtre de visualisation 39, on peut prévoir qu'au niveau des moyens indicateurs de fin de comptage, en particulier au niveau de la seconde partie 52 qui obture complètement la fenêtre 39, il n'y a pas de coopération possible entre les deux disques de comptage 10 et 20. Ceci peut par exemple être réalisé par la suppression d'une dent 22, comme représenté en 29 sur la figure 4. Dans ce cas, même si le premier disque des unités 10 fait encore un tour complet lors d'actionnements ultérieurs du dispositif, le second disque 20 ne sera plus tourné et la fenêtre 39 restera obturée. La rotation du premier disque des unités 10 n'est pas gênante, puisque les chiffres sont invisibles pour l'utilisateur car obturés par les moyens indicateurs de fin de comptage 50 du second disque 20 qui sont disposés au-dessus dans le sens d'observation.

Avantageusement, le compteur comporte en outre une lentille grossissante 40 disposée sur la fenêtre de visualisation 39 pour permettre un grossissement du chiffre inscrit dans ladite fenêtre, et faciliter la visualisation par l'utilisateur du nombre de doses compté ou restant dans le dispositif, selon le type de compteur.

Bien que l'invention ait été décrite en référence à un mode de réalisation particulier de celle-ci, elle n'est en aucun cas limitée à ce mode de réalisation. Au contraire, l'homme du métier peut y apporter toutes modifications souhaitées sans sortir du cadre de la présente invention telle que définie par les revendications annexées.

## Revendications

1. Compteur de doses pour distributeur de produit fluide, comportant deux disques de comptage superposés (10, 20), un premier disque des unités (10) et un second disque des dizaines et/ou centaines (20), chaque disque (10, 20) étant monté rotatif autour d'un axe de rotation (23, 33), lesdits axes de rotation (23, 33) étant parallèles et décalés l'un par rapport à l'autre, **caractérisé en ce que** ledit compteur comporte un couvercle (30), ledit couvercle (30) comportant l'axe de rotation (33) du premier disque (10) et l'axe de rotation (23) du second disque (20).

2. Compteur selon la revendication 1, dans lequel le second disque des dizaines et/ou centaines (20) est au moins partiellement transparent et est disposé au-dessus du premier disque des unités (10) dans le sens d'observation de l'utilisateur.

3. Compteur selon la revendication 1 ou 2, dans lequel le couvercle (30) est disposé au-dessus des disques de comptage (10, 20) dans le sens d'observation de l'utilisateur, ledit couvercle (30) incorporant un logement périphérique (31) recevant le second disque (20) des dizaines et/ou centaines et étant pourvu d'une fenêtre de visualisation (39) pour l'utilisateur, l'axe de rotation (33) du premier disque (10) étant décalé par rapport à l'axe du couvercle (30) formant axe de rotation (23) du second disque (20).

4. Compteur selon l'une quelconque des revendications précédentes, dans lequel les premier et second disques de comptage (10, 20) comportent des moyens anti-retour respectifs (25, 35 ; 16, 36) permettant une rotation seulement dans le sens de comptage.

5. Compteur selon les revendications 3 et 4, dans lequel les moyens anti-retour (16, 36) du premier disque des unités (10) sont formés par un cran radial (36) du couvercle (30) coopérant avec des dents radiales périphériques (16) dudit premier disque (10).

6. Compteur selon les revendications 3 et 4, dans lequel les moyens anti-retour (25, 35) du second disque (20) des dizaines et/ou centaines sont formés par une projection axiale (35) du couvercle (30) coopérant avec des évidements axiaux périphériques (25) dudit second disque (20).

7. Compteur selon l'une quelconque des revendications précédentes, dans lequel le premier disque (10) est entraîné en rotation à chaque actionnement du distributeur de produit fluide et le second disque (20) est entraîné en rotation par le premier disque (10) à chaque tour complet de celui-ci.

8. Compteur selon l'une quelconque des revendications précédentes, dans lequel le compteur est décroissant et compte le nombre de doses restantes dans le distributeur de produit fluide.

9. Compteur selon l'une quelconque des revendications précédentes, dans lequel sont prévus des moyens indicateurs (50) de fin de comptage.

10. Compteur selon la revendication 9, dans lequel lesdits moyens indicateurs (50) sont prévus sur le second disque (20) des dizaines et/ou centaines, et sont réalisés sous la forme d'une partie partiellement (51) et/ou totalement (52) opaque.

11. Distributeur de produit fluide (1), **caractérisé en ce qu'**il comporte un compteur selon l'une quelconque des revendications précédentes.

12. Distributeur selon la revendication 11, comportant un élément d'actionnement (5) adapté à actionner le premier disque de comptage (10) à chaque actionnement dudit distributeur (1).

## Claims

1. A fluid-dispenser dose counter comprising two superposed count disks (10, 20), a first disk (10) that is a units disk, and a second disk (20) that is a tens and/or hundreds disk, each disk (10, 20) being rotably mounted about an axis of rotation (23, 33), said axes of rotation (23, 33) being parallel to and offset from each other, the counter being **characterized in that** it further comprises a cover (30), said cover (30) defining the axis of rotation (33) of the first disk (10) and the axis of rotation (23) of the second disk (20).

2. A counter according to claim 1, in which the tens and/or hundreds, second disk (20) is transparent at least in part, and overlies the units, first disk (10) in the observation direction of the user.

3. A counter according to claim 1 or claim 2, in which the cover (30) overlies the count disks (10, 20) in the observation direction of the user, said cover (30) incorporating a peripheral housing (31) receiving the tens and/or hundreds, second disk (20), and being provided with a viewing window (39) for the user, the axis of rotation (33) of the first disk (10) being offset relative to the axis of the cover (30) which defines the axis of rotation (23) of the second disk (20).

4. A counter according to any preceding claim, in which the first and second count disks (10, 20) include respective anti-return means (16, 36; 25, 35) allowing rotation to take place in the counting direction only.

5. A counter according to claims 3 and 4, in which the anti-return means (16, 36) of the units, first disk (10) are formed by a radially-extending catch (36) of the cover (30) co-operating with peripheral radially-extending teeth (16) of said first disk (10).

6. A counter according to claims 3 and 4, in which the anti-return means (25, 35) of the tens and/or hundreds, second disk (20) are formed by an axially-extending projection (35) inside the cover (30) co-operating with peripheral axially-extending recesses (25) in said second disk (20).

7. A counter according to any preceding claim, in which the first disk (10) is turned each time the fluid dispenser is actuated, and the second disk (20) is turned by the first disk (10) each time said first disk has performed one complete revolution.

8. A counter according to any preceding claim, in which the counter is a down-counter and counts the number of doses remaining in the fluid dispenser.

9. A counter according to any preceding claim, in which end-of-counting indicator means (50) are provided.

10. A counter according to claim 9, in which said indicator means (50) are provided on the tens and/or hundreds, second disk (20), and are made in the form of a portion that is partially and/or completely opaque (51, 52).

11. A fluid dispenser (1), **characterized in that** it includes a counter according to any preceding claim.

12. A dispenser according to claim 11, including an actuator element (5) designed to actuate the first count disk (10) each time said dispenser (1) is actuated.

## Patentansprüche

1. Dosimeter für einen Spender für ein Flüssigkeitsprodukt, aufweisend zwei übereinander angeordnete Zählscheiben (10, 20), eine erste Einerscheibe (10) und eine zweite Zehner- und/oder Hunderterscheibe (20), wobei jede Scheibe (10, 20) drehbar um eine Achse (23, 33) angebracht ist, wobei die Drehachsen (23, 33) parallel verlaufen und in Bezug aufeinander versetzt sind, **dadurch gekennzeichnet, dass** das Dosimeter einen Deckel (30) aufweist, wobei der Deckel (30) die Drehachse (33) der ersten Scheibe (10) und die Drehachse (23) der zweiten Scheibe (20) umfasst.

2. Dosimeter nach Anspruch 1, wobei die zweite Zehner- und/oder Hunderterscheibe (20) zumindest teilweise transparent ist und über der ersten Einerscheibe (10) in Betrachtungsrichtung des Nutzers angeordnet ist.

3. Dosimeter nach Anspruch 1 oder 2, wobei der Deckel (30) über den Zählscheiben (10, 20) in Betrachtungsrichtung des Nutzers angeordnet ist, wobei der Deckel (30) eine periphere Aufnahme (31) enthält, die die zweite Zehner- und/oder Hunderterscheibe (20) aufnimmt und mit einem Sichtfenster (39) für den Nutzer versehen ist, wobei die Drehachse (33) der ersten Scheibe (10) in Bezug auf die Achse des Deckels (30) versetzt ist, die die Drehachse (23) der zweiten Scheibe (20) bildet.

4. Dosimeter nach einem der vorangehenden Ansprüche, wobei die ersten und zweiten Zählscheiben (10, 20) jeweilige Rückstellverhinderungsmittel umfassen, die eine Drehung ausschließlich in Zählrichtung zulassen.

5. Dosimeter nach Anspruch 3 und 4, wobei die Rückstellverhinderungsmittel (16, 36) der ersten Einerscheibe (10) durch einen radialen Einschnitt (36) des Deckels (30) gebildet sind, der mit radialen peripheren Zähnen (16) der ersten Scheibe (10) zusammenwirkt.

6. Dosimeter nach Anspruch 3 und 4, wobei die Rückstellverhinderungsmittel (25, 35) der zweiten Zehner- und/oder Hunderterscheibe (20) durch einen axialen Vorsprung (35) des Deckels (30) gebildet sind, der mit peripheren axialen Eintiefungen (25) der zweiten Scheiben (20) zusammenwirkt.

7. Dosimeter nach einem der vorangehenden Ansprüche, wobei die erste Scheibe (10) bei jeder Betätigung des Fluidproduktspenders drehmäßig mitgenommen wird, und wobei die zweite Scheibe (20) durch die erste Scheibe (10) bei jeder vollständigen Umdrehung derselben drehmäßig mitgenommen wird.

8. Dosimeter nach einem der vorangehenden Ansprüche, wobei das Dosimeter abnehmend anzeigt und die Dosisanzahl zählt, die in dem Fluidproduktspender verbleibt.

9. Dosimeter nach einem der vorangehenden Ansprüche, wobei Anzeigemittel (50) für das Zählungsende vorgesehen sind.

10. Dosimeter nach Anspruch 9, wobei die Anzeigemittel (50) auf der zweiten Zehner- und/oder Hunderterscheibe (20) vorgesehen und in Form eines undurchsichtigen Teils verwirklicht sind.

11. Fluidproduktspender (1), **dadurch gekennzeichnet, dass** er ein Dosimeter nach einem der vorangehenden Ansprüche umfasst.

12. Spender nach Anspruch 11, aufweisend ein Betätigungselement (5), das dazu ausgelegt ist, die erste Zählscheibe (10) bei jeder Betätigung des Spenders (1) zu betätigen.
